# EUROPEAN PATENT APPLICATION

(11) **EP 3 028 572 A1**
(43) Date of publication of application: **08.06.2016**
(21) Application number: 15178731.4
(22) Date of filing: 18.06.2010
(51) Int. Cl.: A01N 43/42, A61K 31/4704, A61K 31/136, A61K 38/16, A61K 45/06

(54) **TREATMENT OF MULTIPLE SCLEROSIS WITH LAQUINIMOD**

(30) Priority: 19.06.2009 US 269070 P
(62) Divisional of application: 10789873.6
(71) Applicant: TEVA PHARMACEUTICAL INDUSTRIES, LTD., 49131 Petah Tiqva (IL)
(72) Inventor: TARCIC, Nora, Modiin (IL); BAR-ZOHAR, Dan, Muttenz, 4132 (CH); KOFLER, Dina, Kadima-Zoran (IL)
(74) Representative: D Young & Co LLP

(57) **Abstract**

The subject invention provides for methods of reducing the relapse rate and/or reducing the accumulation of physical disability in a relapsing-remitting multiple sclerosis human patient, the method comprising orally administering to the patient a daily dose of 0.6 mg laquinimod.

The subject invention also provides for pharmaceutical oral unit dosage forms of 0.6 mg laquinimod for use in reducing the relapse rate and/or for use in reducing the accumulation of physical disability in a relapsing-remitting multiple sclerosis human patient.

## Description

This application clauses the benefit of U.S. Provisional Application No. 61/269,070, filed June 19, 2009, the entire content of which is hereby incorporated by reference herein.

Throughout this application, various publications are referred to by first author and year of publication. Full citations for these publications are presented in a References section immediately before the claims. Disclosures of the publications cited in the References section in their entireties are hereby incorporated by reference into this application in order to more fully describe the state of the art as of the date of the invention described herein.

### Background

Multiple Sclerosis (MS) is a neurological disease affecting more than 1 million people worldwide. It is the most common cause of neurological disability in young and middle-aged adults and has a major physical, psychological, social and financial impact on subjects and their families, friends and bodies responsible for health care. (EMEA Guideline, 2006)

It is generally assumed that MS is mediated by some kind of autoimmune process possibly triggered by infection and superimposed upon a genetic predisposition. It is a chronic inflammatory condition that damages the myelin of the Central Nervous System (CNS). The pathogenesis of MS is characterized by the infiltration of autoreactive T-cells from the circulation directed against myelin antigens into the CNS. (Bjartmar, 2002) In addition to the inflammatory phase in MS, axonal loss occurs early in the course of the disease and can be extensive over time, leading to the subsequent development of progressive, permanent, neurologic impairment and, frequently, severe disability. (Neuhaus, 2003) Symptoms associated with the disease include fatigue, spasticity, ataxia, weakness, bladder and bowel disturbances, sexual dysfunction, pain, tremor, paroxysmal manifestations, visual impairment, psychological problems and cognitive dysfunction. (EMEA Guideline, 2006)

Various MS disease stages and/or types are described in Multiple Sclerosis Therapeutics. (Duntiz, 1999) Among them, relapsing-remitting multiple sclerosis (RRMS) is the most common form at the time of initial diagnosis. Many subjects with RRMS have an initial relapsing-remitting course for 5-15 years, which then advances into the secondary progressive MS (SPMS) disease course. Relapses result from inflammation and demyelination, whereas restoration of nerve conduction and remission is accompanied by resolution of inflammation, redistribution of sodium channels on demyelinated axons and remyelination. (Neuhaus, 2003; Noseworthy, 2000)

In April 2001, an international panel in association with the National MS Society of America recommended diagnostic criteria for multiple sclerosis. These criteria became known as the McDonald Criteria. The McDonald Criteria make use of MRI techniques and are intended to replace the Poser Criteria and the older Schumacher Criteria. (McDonald, 2001) The McDonald Criteria was revised in March 2005 by an international panel. (Polman, 2005)

Intervention with disease-modifying therapy at relapsing stages of MS is suggested to reduce and/or prevent accumulating neurodegeneration. (Hohlfeld, 2000; De Stefano, 1999) There are currently six disease-modifying medications approved for use in relapsing MS (RMS), which includes RRMS and SPMS. (The Disease Modifying Drug Brochure, 2006) These include interferon beta 1-a (Avonex® and Rebif®), interferon beta 1-b (Betaseron®), glatiramer acetate (Copaxone®), mitoxantrone (Novantrone®) and natalizumab (Tysabri®). Most of them are believed to act as immunomodulators. Mitoxantrone and natalizumab are believed to act as immunesuppressants. However, the mechanisms of action of each have been only partly elucidated. Immunosuppressants or cytotoxic agents are used in some subjects after failure of conventional therapies. However, the relationship between changes of the immune response induced by these agents and the clinical efficacy in MS is far from settled. (EMEA Guideline, 2006)

Other therapeutic approaches include symptomatic treatment which refers to all therapies applied to improve the symptoms caused by the disease (EMEA Guideline, 2006) and treatment of acute relapses with corticosteroids. While steroids do not affect the course of MS over time, they can reduce the duration and severity of attacks in some subjects.

### Laquinimod

Laquinimod sodium is a novel synthetic compound with high oral bioavailability, which has been suggested as an oral formulation for the treatment of MS. (Polman, 2005; Sandberg-Wollheim, 2005)

Studies have shown laquinimod to reduce development of active MRI lesions in relapsing MS. (Polman, 2005) However, the clinical significance of MRI brain lesion reduction alone is still unsettled. Although MRI lesions are used as the primary outcome measure in some studies, others have suggested that correlation between MRI abnormalities and clinical disease activity in patients with RRMS is weak and that such measurement should be used as secondary outcomes rather than as surrogate markers of clinical responses. (Rudick, 1999; Miki, 1999; Barkhof, 1999) Further, according to pharmaceutical regulatory bodies such as the European Medicines Agency (EMEA), the correlation between MRI results and clinical outcomes has not been proved strong enough so as to accept MRI results as validated surrogate endpoint in pivotal studies. Therefore, according to the EMEA, the relevant efficacy parameter for clinical trials is the accumulation of disability and relapse rate (for RRMS). (EMEA Guideline, 2006) Thus, relapse rate and progression of disability are the currently accepted indicators of the effectiveness of a treatment for RRMS, but these have not previously been established for laquinimod.

The EMEA MS clinical trials guideline further states that the annual relapse rate in RRMS is usually low and that, generally, progression of disability takes years. Consequently, confirmatory studies with products intended to modify the course of the disease should be large scale and long enough to have a substantial proportion of patients suffering relapses or showing progression of disability. Two years is considered the minimum duration to demonstrate efficacy. (EMEA Guideline, 2006)

Furthermore, existing literatures reached different conclusions as to the effective dose of laquinimod for the treatment of MS. The 0.3mg/day oral dose was shown to reduce development of active MRI lesions in relapsing MS (which includes RRMS and SPMS) in one study (Polman, 2005), while another study showed the same dose to have neither MRI nor clinical effect as compared to placebo. (Comi, 2007)

### Summary of the Invention

Disclosed herein is finding that administration of a daily oral dose of 0.6 mg laquinimod reduces relapse rate and progression of EDSS as well as reduce MRI-monitored disease activity in relapsing-remitting multiple sclerosis.

The subject invention provides a method of reducing the relapse rate in a relapsing-remitting multiple sclerosis human patient, the method comprising orally administering to the patient laquinimod or a pharmaceutically acceptable salt thereof at a daily dose of 0.6 mg laquinimod so as to thereby reduce the relapse rate.

The subject invention also provides a method of reducing the accumulation of physical disability in a relapsing-remitting multiple sclerosis human patient, the method comprising orally administering to the patient laquinimod or a pharmaceutically acceptable salt thereof at a daily dose of 0.6 mg laquinimod so as to thereby reduce the accumulation of physical disability.

The subject invention also provides a pharmaceutical oral unit dosage form of 0.6 mg laquinimod for use in reducing the relapse rate in a relapsing-remitting multiple sclerosis human patient.

The subject invention also provides a pharmaceutical oral unit dosage form of 0.6 mg laquinimod for use in reducing the accumulation of physical disability in a relapsing-remitting multiple sclerosis human patient.

### Detailed Description of the Invention

The subject invention provides a method of reducing the relapse rate in a relapsing-remitting multiple sclerosis human patient, the method comprising orally administering to the patient laquinimod or a pharmaceutically acceptable salt thereof at a daily dose of 0.6 mg laquinimod so as to thereby reduce the relapse rate.

In one embodiment, the relapse rate is reduced by at least 30%. In another embodiment, the relapse rate is reduced by at least 70%.

In one embodiment, the laquinimod is administered in the form of laquinimod sodium.

In one embodiment, the laquinimod is administered as monotherapy for relapsing-remitting multiple sclerosis. In another embodiment, the laquinimod is administered as adjunct therapy with an other relapsing-remitting multiple sclerosis treatment. In yet another embodiment, the other relapsing-remitting multiple sclerosis treatment is administration of interferon beta 1-a, interferon beta 1-b, glatiramer acetate, mitoxantrone or natalizumab.

In one embodiment, the administration is for a period of greater than 24 weeks.

The subject invention also provides a method of reducing the accumulation of physical disability in a relapsing-remitting multiple sclerosis human patient, the method comprising orally administering to the patient laquinimod or a pharmaceutically acceptable salt thereof at a daily dose of 0.6 mg laquinimod so as to thereby reduce the accumulation of physical disability.

In one embodiment, the accumulation of physical disability is assessed by the time to confirmed disease progression as measured by Kurtzke Expanded Disability Status Scale (EDSS) score.

In one embodiment, the patient had an EDSS score of 0-5.5 prior to administration of laquinimod. In another embodiment, confirmed disease progression is a 1 point increase of the EDSS score.

In one embodiment, the patient had an EDSS score of 5.5 or greater prior to administration of laquinimod. In another embodiment, confirmed disease progression is a 0.5 point increase of the EDSS score.

In one embodiment, time to confirmed disease progression is increased by 20-60%. In another embodiment, time to confirmed disease progression is increased by 50%.

In one embodiment, the laquinimod is administered in the form of laquinimod sodium.

In one embodiment, the laquinimod is administered as monotherapy for relapsing-remitting multiple sclerosis. In another embodiment, the laquinimod is administered as adjunct therapy with an other relapsing-remitting multiple sclerosis treatment. In yet another embodiment, the other relapsing-remitting multiple sclerosis treatment is administration of interferon beta 1-a, interferon beta 1-b, glatiramer acetate, mitoxantrone or natalizumab.

In one embodiment, the administration is for a period of greater than 24 weeks.

The subject invention also provides a pharmaceutical oral unit dosage form of 0.6 mg laquinimod for use in reducing the relapse rate in a relapsing-remitting multiple sclerosis human patient.

The subject invention also provides a pharmaceutical oral unit dosage form of 0.6 mg laquinimod for use in reducing the accumulation of physical disability in a relapsing-remitting multiple sclerosis human patient.

For the foregoing embodiments, each embodiment disclosed herein is contemplated as being applicable to each of the other disclosed embodiment.

A pharmaceutically acceptable salt of laquinimod as used in this application includes lithium, sodium, potassium, magnesium, calcium, manganese, copper, zinc, aluminum and iron. Salt formulations of laquinimod and the process for preparing the same are described, e.g., in U.S. Patent Application Publication No. 2005/0192315 and PCT International Application Publication No. WO 2005/074899, which are hereby incorporated by reference into this application.

A dosage unit may comprise a single compound or mixtures of compounds thereof. A dosage unit can be prepared for oral dosage forms, such as tablets, capsules, pills, powders, and granules.

Laquinimod can be administered in admixture with suitable pharmaceutical diluents, extenders, excipients, or carriers (collectively referred to herein as a pharmaceutically acceptable carrier) suitably selected with respect to the intended form of administration and as consistent with conventional pharmaceutical practices. The unit will be in a form suitable for oral administration. Laquinimod can be administered alone but is generally mixed with a pharmaceutically acceptable carrier, and co-administered in the form of a tablet or capsule, liposome, or as an agglomerated powder. Examples of suitable solid carriers include lactose, sucrose, gelatin and agar. Capsule or tablets can be easily formulated and can be made easy to swallow or chew; other solid forms include granules, and bulk powders. Tablets may contain suitable binders, lubricants, diluents, disintegrating agents, coloring agents, flavoring agents flow-inducing agents, and melting agents.

Specific examples of the techniques, pharmaceutically acceptable carriers and excipients that may be used to formulate oral dosage forms of the present invention are described, e.g., in U.S. Patent Application Publication No. 2005/0192315, PCT International Application Publication Nos. WO 2005/074899, WO 2007/047863, and 2007/146248.

General techniques and compositions for making dosage forms useful in the present invention are described-in the following references: 7 Modern Pharmaceutics, Chapters 9 and 10 (Banker & Rhodes, Editors, 1979); Pharmaceutical Dosage Forms: Tablets (Lieberman et al., 1981); Ansel, Introduction to Pharmaceutical Dosage Forms 2nd Edition (1976); Remington's Pharmaceutical Sciences, 17th ed. (Mack Publishing Company, Easton, Pa., 1985); Advances in Pharmaceutical Sciences (David Ganderton, Trevor Jones, Eds., 1992); Advances in Pharmaceutical Sciences Vol 7. (David Ganderton, Trevor Jones, James McGinity, Eds., 1995); Aqueous Polymeric Coatings for Pharmaceutical Dosage Forms (Drugs and the Pharmaceutical Sciences, Series 36 (James McGinity, Ed., 1989); Pharmaceutical Particulate Carriers: Therapeutic Applications: Drugs and the Pharmaceutical Sciences, Vol 61 (Alain Rolland, Ed., 1993); Drug Delivery to the Gastrointestinal Tract (Ellis Horwood Books in the Biological Sciences. Series in Pharmaceutical Technology; J. G. Hardy, S. S. Davis, Clive G. Wilson, Eds.); Modern Pharmaceutics Drugs and the Pharmaceutical Sciences, Vol. 40 (Gilbert S. Banker, Christopher T. Rhodes, Eds.). These references in their entireties are hereby incorporated by reference into this application.

Tablets may contain suitable binders, lubricants, disintegrating agents, coloring agents, flavoring agents, flow-inducing agents, and melting agents. For instance, for oral administration in the dosage unit form of a tablet or capsule, the active drug component can be combined with an oral, non-toxic, pharmaceutically acceptable, inert carrier such as lactose, gelatin, agar, starch, sucrose, glucose, methyl cellulose, dicalcium phosphate, calcium sulfate, mannitol, sorbitol, microcrystalline cellulose and the like. Suitable binders include starch, gelatin, natural sugars such as glucose or beta-lactose, corn starch, natural and synthetic gums such as acacia, tragacanth, or sodium alginate, povidone, carboxymethylcellulose, polyethylene glycol, waxes, and the like. Lubricants used in these dosage forms include sodium oleate, sodium stearate, sodium benzoate, sodium acetate, sodium chloride, stearic acid, sodium stearyl fumarate, talc and the like. Disintegrators include, without limitation, starch, methyl cellulose, agar, bentonite, xanthan gum, croscarmellose sodium, sodium starch glycolate and the like.

### Terms

As used herein, and unless stated otherwise, each of the following terms shall have the definition set forth below.

A "dose of 0.6 mg laquinimod" means the amount of laquinimod acid in a preparation is 0.6 mg, regardless of the form of the preparation. Thus, when in the form of a salt, e.g. a laquinimod sodium salt, the weight of the salt form necessary to provide a dose of 0.6 mg laquinimod would be greater than 0.6 mg due to the presence of the additional salt ion.

"Relapsing-Remitting Multiple Sclerosis" or "RRMS" is characterized by clearly defined acute attacks with full recovery or with sequelae and residual deficit upon recovery, where periods between disease relapses are characterized by a lack of disease progression. (Lublin, 1996)

"Confirmed Relapse" is defined as the appearance of one or more new neurological abnormalities or the reappearance of one or more previously observed neurological abnormalities wherein the change in clinical state lasts at least 48 hours and is immediately preceded by an improving neurological state of at least thirty (30) days from onset of previous relapse. This criterion is different from the clinical definition of relapse which requires only 24 hours duration of symptoms. (EMEA Guideline, 2006) Since "in study" relapse definition must be supported by an objective neurological evaluation as discussed below, a neurological deficit must sustain long enough to eliminate pseudo-relapses.

An event is a relapse only when the subject's symptoms are accompanied by observed objective neurological changes, consistent with at least one of the following: an increase of at least 0.5 in the EDSS score as compared to the previous evaluation, an increase of one grade in the score of 2 or more of the 7 FS functions as compared to the previous evaluation, or an increase of 2 grades in the score of one FS as compared to the previous evaluation.

In addition, the subject must not be undergoing any acute metabolic changes such as fever or other medical abnormality. A change in bowel/bladder function or in cognitive function must not be entirely responsible for the changes in EDSS or FS scores.

"Relapse Rate" is the number of confirmed relapses per unit time. "Annualized relapse rate" is the mean value of the number of confirmed relapses of each patient multiplied by 365 and divided by the number of days that patient is on the study drug.

"Expanded Disability Status Scale" or "EDSS" is a rating system that is frequently used for classifying and standardizing the condition of people with multiple sclerosis. The score ranges from 0.0 representing a normal neurological exam to 10.0 representing death due to MS. The score is based upon neurological testing and examination of functional systems (FS), which are areas of the central nervous system which control bodily functions. The functional systems are: Pyramidal (ability to walk), Cerebellar (coordination), Brain stem (speech and swallowing), Sensory (touch and pain), Bowel and bladder functions, Visual, Mental, and Other (includes any other neurological findings due to MS). (Kurtzke JF, 1983)

A "confirmed progression" of EDSS, or "confirmed disease progression" as measured by EDSS score is defined as a 1 point increase from baseline EDSS if baseline EDSS was between 0 and 5.0, or a 0.5 point increase if baseline EDSS was 5.5. In order to be considered a confirmed progression, the change (either 1 point or 0.5 points) must be sustained for at least 3 months. In addition, confirmation of progression cannot be made during a relapse.

"Adverse event" or "AE" means any untoward medical occurrence in a clinical trial subject administered a medicinal product and which does not have a causal relationship with the treatment. An adverse event can therefore be any unfavorable and unintended sign including an abnormal laboratory finding, symptom, or diseases temporally associated with the use of an investigational medicinal product, whether or not considered related to the investigational medicinal product.

"Ambulation Index" or "AI" is a rating scale developed by Hauser et al. to assess mobility by evaluating the time and degree of assistance required to walk 25 feet. Scores range from 0 (asymptomatic and fully active) to 10 (bedridden). The patient is asked to walk a marked 25-foot course as quickly and safely as possible. The examiner records the time and type of assistance (e.g., cane, walker, crutches) needed. (Hauser, 1983)

"EQ-5D" is a standardized questionnaire instrument for use as a measure of health outcome applicable to a range of health conditions and treatments. It provides a simple descriptive profile and a single index value for health status that can be used in the clinical and economic evaluation of health care as well as population health surveys. EQ-5D was developed by the "EuroQoL" Group which comprises a network of international, multilingual, multidisciplinary researchers, originally from seven centers in England, Finland, the Netherlands, Norway and Sweden. The EQ-5D questionnaire is in the public domain and can be obtained from EuroQoL.

"Gd-enhancing lesion" refers to lesions that result from a breakdown of the blood-brain barrier, which appear in contrast studies using gandolinium contrast agents. Gandolinium enhancement provides information as to the age of a lesion, as Gd-enhancing lesions typically occur within a six week period of lesion formation.

"Magnetization Transfer Imaging" or "MTI" is based on the magnetization interaction (through dipolar and/or chemical exchange) between bulk water protons and macromolecular protons. By applying an off resonance radio frequency pulse to the macromolecular protons, the saturation of these protons is then transferred to the bulk water protons. The result is a decrease in signal (the net magnetization of visible protons is reduced), depending on the magnitude of MT between tissue macromolecules and bulk water. "MT" or "Magnetization Transfer" refers to the transfer of longitudinal magnetization from the hydrogen nuclei of water that have restricted motion to the hydrogen nuclei of water that moves with many degrees of freedom. With MTI, the presence or absence of macromolecules (e.g. in membranes or brain tissue) can be seen. (Mehta, 1996; Grossman, 1994)

"Magnetization Resonance Spectroscopy" or "MRS" is a specialized technique associated with magnetic resonance imaging (MRI). MRS is used to measure the levels of different metabolites in body tissues. The MR signal produces a spectrum of resonances that correspond to different molecular arrangements of the isotope being "excited". This signature is used to diagnose certain metabolic disorders, especially those affecting the brain, (Rosen, 2007) as well as to provide information on tumor metabolism. (Golder, 2007)

"Modified Fatigue Impact Scale" or "MFIS" is a validated specific subject-reported outcome measure developed to evaluate the impact of fatigue on the lives of people with MS. This instrument provides an assessment of the effects of fatigue in terms of physical, cognitive, and psychosocial functioning. The full-length MFIS consists of 21 items while the abbreviated version has 5 items.

### (Fisk et al, 1994)

"MS Functional Composite" or "MSFC" is a clinical outcome measure for MS. The MSFC comprises quantitative functional measures of three key clinical dimensions of MS: leg function/ambulation, arm/hand function, and cognitive function. Scores on component measures are converted to standard scores (z-scores), which are averaged to form a single MSFC score. (Fischer, 1999)

"SF-36" is a multi-purpose, short-form health survey with 36 questions which yields an 8-scale profile of functional health and well-being scores as well as psychometrically-based physical and mental health summary measures and a preference-based health utility index. It is a generic measure, as opposed to one that targets a specific age, disease, or treatment group. The survey is developed by and can be obtained from QualityMetric, Inc. of Providence, RI.

"T1-weighted MRI image" refers to an MR-image that emphasizes T1 contrast by which lesions may be visualized. Abnormal areas in a T1-weighted MRI image are "hypointense" and appear as dark spots. These spots are generally older lesions.

"T2-weighted MRI image" refers to an MR-image that emphasizes T2 contrast by which lesions may be visualized. T2 lesions represent new inflammatory activity.

A "pharmaceutically acceptable carrier" refers to a carrier or excipient that is suitable for use with humans and/or animals without undue adverse side effects (such as toxicity, irritation, and allergic response) commensurate with a reasonable benefit/risk ratio. It can be a pharmaceutically acceptable solvent, suspending agent or vehicle, for delivering the instant compounds to the subject.

It is understood that where a parameter range is provided, all integers within that range, and tenths thereof, are also provided by the invention. For example, "5-10%" includes 5.0%, 5.1%, 5.2%, 5.3%, 5.4% etc. up to 10.0%.

This invention will be better understood by reference to the Experimental Details which follow, but those skilled in the art will readily appreciate that the specific experiments detailed are only illustrative of the invention as described more fully in the clauseswhich follow thereafter.

### Experimental Details

### EXAMPLE 1: Clinical Trial (Phase III) - Assessment of Oral Laquinimod in Preventing Progression of MS

A multinational, multicenter (approximately 175 centers), randomized, double-blinded, parallel-group, placebo-controlled clinical trial ("ALLEGRO") is conducted to evaluate the safety, tolerability and efficacy of daily oral administration of laquinimod 0.6 mg in subjects with relapsing remitting multiple sclerosis (RRMS).

### Study Title

A multinational, multicenter, randomized, double-blind, parallel-group, placebo-controlled study, to evaluate the safety, tolerability and efficacy of daily oral administration of laquinimod 0.6 mg in subjects with relapsing remitting multiple sclerosis (RRMS).

### Study Duration

Screening phase: 1 month.

Double blind treatment phase: 24 months of once-daily oral administration of daily dose of 0.6 mg laquinimod or matching placebo.

Upon blinded variance and power reassessment of the population progression (planned prior to first subject completes the 20 months of treatment), the double blind study duration may be extended to 30 months. This is planned in order to enhance the statistical power to detect the effect of laquinimod on disability accumulation. The recommendation to extend the study duration is based on a pre-defined rule.

### Study Population

Relapsing Remitting Multiple Sclerosis (RRMS).

### Study Design

Eligible subjects are equally randomized into one of the following treatment arms:
1. Laquinimod capsules 0.6 mg. The 0.6 mg laquinimod is administered as laquinimod capsules which contains 0.6 mg of Laquinimod Acid per capsule with meglumine.
2. Matching placebo capsules.

Subjects are evaluated at study sites for 12 scheduled visits of the double blind phase at months: -1 (screening), 0 (baseline), 1, 2, 3, 6, 9, 12, 15, 18, 21 and 24 (termination/early discontinuation). In case of the 6 months extended study, subjects are evaluated at study sites at months 27 and 30 (termination/early discontinuation of extended study), in this case month 24 is a regular scheduled visit.

Subjects successfully completing the study are offered the opportunity to enter into a 1-year open label extension.

The following assessments are performed at specified time points:
1. Vital signs are measured at each study visit.
2. A physical examination is performed at months -1 (screening), 0 (baseline) 1, 3, 6, 12, 18 and 24 (termination/early discontinuation core study). In case of the 6 months extended study, additional examination is performed at month 30 (termination/early discontinuation of extended study).
3. The following safety clinical laboratory tests are performed:
   a. Complete blood count (CBC) with differential - at all scheduled visits. A reticulocyte count is added to the CBC at months 0 (baseline) and 24/30 (termination/early discontinuation).
   b. Serum chemistry (including electrolytes, liver enzymes, direct and total bilirubin and pancreatic amylase and CPK), and urinalysis - at all scheduled visits.
   c. A rapid urine β-hCG test is performed in women of child-bearing potential at baseline (month 0) and at each scheduled study visit thereafter (at site).
   d. β-hCG in women of child-bearing potential is performed at all scheduled visits.
   e. Starting after visit Month 3 a rapid urine β-hCG test is performed in women of child-bearing potential every 28 (±2) days. The subject is contacted by telephone within 72 hours after the test is scheduled to be performed and asked specific questions regarding the test. In case of suspected pregnancy (positive urine β-hCG test result), the caller makes sure that the study drug has been discontinued and the subject is instructed to arrive at the site as soon as possible with all study drugs.
4. Markers of inflammation (serum conventional C-reactive protein and fibrinogen) - at screening, baseline and all scheduled visits thereafter.
5. During the first 3 months periodical phone calls are placed by the site personnel every two weeks. A list of predefined questions relating to signs/symptoms suggestive of vascular thrombosis is presented to the subjects.
6. ECG is performed at months -1 (screening; additional recording, up to 30 minutes apart is performed if QT_{c} is less than 450 msec), (baseline; three recordings, 15 minutes apart), 1, 2, 3, 6, 12, 18 and 24 (termination/early discontinuation). In case of the 6 months extended study, ECG is performed at month 30 (termination/early discontinuation of the extended study).
7. Chest X-ray is performed at months -1 (screening), (if not performed within 7 months prior to the screening visit).
8. Adverse Events (AEs) are monitored throughout the study.
9. Concomitant medications are monitored throughout the study.
10. Neurological evaluations, including Expanded Disability Status Scale (EDSS), 25 foot walk test/Ambulation Index (AI), Functional systems (FS) are performed at months -1 (screening), 0 (baseline) and every 3 months during the study and the extended study period.
11. MS functional Composite (MSFC) is assessed at months -1 (screening) (three practices for training purposes only), at month 0 (baseline), 6, 12, 18 and 24 (termination/early discontinuation). In case of the 6 months extended study, the last MSFC is performed at months 30 (termination/early discontinuation of the extended study).
12. Subject-reported fatigue is assessed by the Modified Fatigue Impact Scale (MFIS) at months 0, 6, 12, 18, and 24 (termination/early discontinuation). In case of the 6 months extended study, additional MFIS is performed at month 30 (termination/early discontinuation of the extended study).
13. The general health status is assessed by the EuroQoL (EQ5D) questionnaire at month 0 (baseline) and month 24 (termination/early discontinuation of the study). In case of the 6 months extended study, the last EuroQoL (EQ5D) is performed at month 30 (termination/early discontinuation of the extended study) instead of month 24.
14. The general health status is assessed by the Short-Form general health survey (SF-36) subject-reported questionnaire at month 0 (baseline) and every 6 months thereafter, until termination/early discontinuation.
15. The subject undergoes 5 assessments of binocular low-contrast visual acuity using the 100%, 2.5% and 1.25% contrast level charts [Sloan letter or Tumbling-E] in each assessment, at months 0 (baseline), 6, 12, 18 and 24 (termination/early discontinuation). In case of extending the study for 6 months, additional binocular low-contrast visual acuity assessment is performed at month 30 (termination/early discontinuation of the extended study).
16. Serum samples are collected from all subjects in order to investigate the potential mechanism of action of laquinimod and additional biomarkers of inflammation and potential biomarkers of MS disease at months: 0, 1, 12 and 24. In case of extending the study for 6 months the last serum sample is performed at month 30 (termination/early discontinuation of the extended study) instead of month 24.
17. The subjects undergoes 3 MRI scans at months 0 (baseline), 12 and 24 (termination/early discontinuation). In case of the 6 months extended study, an additional MRI is performed at month 30 (termination/early discontinuation of the extended study).
18. Population PK study (PPK): Blood samples for PPK evaluation is collected from all subjects at months 1, 12 and 24. In case of extending the study for 6 months the last PPK evaluation is performed at month 30 (termination/early discontinuation of the extended study) instead of month 24.
19. Relapses are confirmed/monitored through the study. Since the "in study" relapse definition must be supported by an objective neurological evaluation, a neurological deficit must sustain long enough to eliminate pseudo-relapses. Therefore, in Experiment 1, a relapse is the appearance of one or more new neurological abnormalities or the reappearance of one or more previously observed neurological abnormalities wherein the change in clinical state lasts at least 48 hours and is immediately preceded by an improving neurological state of at least thirty (30) days from onset of previous relapse.
20. The allowed treatment for a relapse is intravenous Methylprednisolone 1 gr/day for up to 5 consecutive days.

### Re-consent criteria

Upon a confirmed diagnosis of MS relapse, (as defined in the protocol) or an increase in EDSS in ≥2.0 points, sustained for ≥3 months, the following actions are taken:
1. The subject is reminded of the current available MS medications and the opportunity to terminate the study as written in the informed consent form.
2. The subject is requested to re-sign an informed consent form if he/she chooses to continue to participate in the study, in the same treatment assignment.

Safety stopping rules are set in place for the management of: 1) elevated liver enzymes, 2) inflammatory events, 3) thrombotic events and 4) pancreatitis.

### Ancillary studies:

1. Frequent MRI (selected countries and sites only): The cumulative number of T₁-Gd enhancing lesions taken from scans obtained at months 0, 3, 6, 12, and 24, and in case the study is be extended, 30. Additional MRIs for the ancillary study are performed at months 3 and 6.
2. Magnetization Transfer (MT) (selected countries and sites only): the change from baseline to month 12 and 24/30 months in magnetization transfer MRI. MT is assessed at months 0 (baseline), 12 and 24 (termination/early discontinuation). In case of the 6 months extended study, the last MT is performed at month 30 (termination/early discontinuation of the extended study) instead of month 24.
3. Magnetization Resonance Spectroscopy (MRS) (selected countries and sites only): Change from baseline to 24/30 in Magnetic Resonance Spectroscopy (NAAS: Cr ratio in lesions, normally-appearing white matter). MRS is assessed at months 0 (baseline), and 24 (termination/early discontinuation). In case of the 6 months extended study, the last MRS is performed at month 30 (termination/early discontinuation of the extended study) instead of month 24.
4. Pharmacogenetic (PGx) assessment: Blood samples for PGx parameters are collected from all subjects at screening.
5. Brain atrophy, as defined by the percentage of change from one scan to the subsequent scan in brain volume, in addition to the measurements done in the main study (Frequent MRI Cohort).
6. Whole blood and serum samples (selected countries and sites only) are collected for evaluation of the immunological response to treatment with laquinimod and further investigation of the potential mechanism of action. Whole blood samples are collected at months: 0, 1, 3, 6, 12 and 24. Serum samples are collected at month: 0, 1, 6, 12 and 24 (even if the study is extended to month 30).
7. Relationship between PGx and response to laquinimod in terms of clinical, MRI and safety parameters.

### Number of Subjects

Approximately 1000 subjects.

### Inclusion/Exclusion Criteria

### Inclusion Criteria

1. Subjects must have a confirmed and documented diagnosis as defined by the Revised McDonald Criteria (Polman, 2005), with relapsing-remitting disease course.
2. Subjects must be ambulatory with converted Kurtzke EDSS score of 0-5.5.
3. Subjects must be in a stable neurological condition and free of corticosteroid treatment [intravenous (iv), intramuscular (im) and/or per os (po)] 30 days prior to screening (month -1).
4. Subjects must have experienced one of the following:
   a. At least one documented relapse in the 12 months prior to screening.
   b. At least two documented relapses in the 24 months prior to screening.
   c. One documented relapse between 12 and 24 months prior to screening with at least one documented T1-Gd enhancing lesion in an MRI performed within 12 months prior to screening.
5. Subjects must be between 18 and 55 years of age, inclusive.
6. Subjects must have disease duration of at least 6 months (from the first symptom) prior to screening.
7. Women of child-bearing potential must practice an acceptable method of birth control. Acceptable method of birth control in this study include: surgical sterilization, intrauterine devices, oral contraceptive, contraceptive patch, long-acting injectable contraceptive, partner's vasectomy or double barrier method (condom or diaphragm with spermicide).
8. Subjects must be able to sign and date a written informed consent prior to entering the study.
9. Subjects must be willing and able to comply with the protocol requirements for the duration of the study.

### Exclusion Criteria

1. Subjects with progressive forms of MS.
2. An onset of relapse, unstable neurological condition or any treatment with corticosteroids [(iv), intramuscular (im) and/or per os (po)] or ACTH between months -1 (screening) and 0 (baseline).
3. Use of experimental or investigational drugs, and/or participation in drug clinical studies within the 6 months prior to screening.
4. Use of immunosuppressive including mitoxantrone (Novantrone®) or cytotoxic agents within 6 months prior to screening visit.
5. Previous use of any one of the following: natalizumab (Tysabri®), caldribine, laquinimod.
6. Previous treatment with glatiramer acetate (copaxone®) Interferon-β (either 1a or 1b) or IVIG within 2 months prior to screening visit.
7. Systemic corticosteroid treatment of ≥30 consecutive days duration within 2 months prior to screening visit.
8. Previous total body irradiation or total lymphoid irradiation.
9. Previous stem cell treatment, autologous bone marrow transplantation or allogenic bone marrow transplantation.
10. A known history of tuberculosis.
11. Acute infection two weeks prior to baseline visit.
12. Major trauma or surgery two weeks prior to baseline.
13. Use of inhibitors of CYP3A4 within 2 weeks prior to baseline visit (1 month for fluoxetine).
14. Use of amiodarone within 2 years prior to screening visit.
15. Pregnancy or breastfeeding.
16. A ≥3xULN serum elevation of either ALT or AST at screening.
17. Serum direct bilirubin which is ≥2xULN at screening.
18. A QTc interval which is 450 msec (according to machine output) obtained from:
   a. Two ECG recordings at screening visit, or
   b. The mean value calculated from 3 baseline ECG recordings.
19. Subjects with clinically significant or unstable medical or surgical condition that would preclude safe and complete study participation, as determined by medical history, physical examination, ECG, laboratory tests or chest X-ray. Such conditions may include:
   a. A cardiovascular or pulmonary disorder that cannot be well-controlled by standard treatment permitted by the study protocol.
   b. A gastrointestinal disorder that may affect the absorption of study medication.
   c. Renal or metabolic diseases.
   d. Any form of chronic liver disease.
   e. Known human immunodeficiency virus (HIV posibtive status.
   f. A family history of Long- QT syndrome.
   g. A history of drug and/or alcohol abuse.
   h. Major psychiatric disorder.
20. A known history of sensitivity to Gd.
21. Inability to successfully undergo MRI scanning.
22. Known drug hypersensitivity that would preclude administration of laquinimod, such as hypersensitivity to: mannitol, meglumine or sodium stearyl fumarate.

### Route and Dosage Form

0.6mg arm: one capsule containing 0.6 mg laquinimod is administered orally once daily. The 0.6 mg laquinimod capsule contains 0.6 mg of Laquinimod Acid per capsule with meglumine.

The 0.6 mg laquinimod capsule is manufactured according to the method disclosed in PCT International Application Publication No. WO/2007/146248, published December 21, 2007 (see, page 10, line 5 to page 11, line 3).

Matching placebo for laquinimod arm: one capsule is administered once daily.

### Outcome Measures

### Primary Outcome Measure

The number of confirmed relapses during the double blind study period.

### Secondary Outcome Measures

1. Accumulation of physical disability measured by the time to confirmed progression of EDSS during the study period (A confirmed progression of EDSS is defined as a 1 point increase from baseline on EDSS score if baseline EDSS was between 0 and 5.0, or a 0.5 point increase if the baseline EDSS was 5.5, confirmed 3 months later. Progression cannot be confirmed during a relapse).
2. Disability, as assessed by the MSFC score at the end of the treatment period (month 24/30).
3. The cumulative number of new T2 lesions on scans taken on months 12 and 24 (and 30 in the case of the 6 months extended study).
4. The cumulative number of enhancing lesions on T1-weighted images taken on months 12 and 24 (and 30 in case of extending the study for 6 months.)

### Safety and Tolerability Outcome Measures

1. Adverse events.
2. Vital signs.
3. ECG findings.
4. Clinical laboratory parameters.
5. Proportion of subjects (%) who prematurely discontinued from the study, reason of discontinuation and the time to withdrawal.
6. Proportion of subjects (%) who prematurely discontinued form the study due to AEs and the time to withdrawal.

### Additional Exploratory Endpoints

The following assessments are performed in an exploratory manner:
1. To assess the cumulative number of new hypointense lesions on enhanced T₁ scans at months 12 and 24 (and 30 in case of extending the study for 6 months).
2. Subject-reported fatigue as assessed by the Modified Fatigue Impact Scale (MFIS).
3. General health status by the EuroQoL (EQ5D) questionnaire.
4. The general health status assessed by the Short-Form general health survey (SF-36) subject-reported questionnaire.
5. The time to the first confirmed relapse during the study period.
6. The rate of confirmed relapses during the study period, requiring hospitalization and/or IV steroids.
7. The proportion of relapse free subjects.
8. The change in T₂-lesion volume as defined by the change from baseline to month 12, from month 12 to month 24/30 and from baseline to month 24/30.
9. The change in T₁-hypointense lesion volume as defined by the change from baseline to month 12, from month 12 to month 24/30 and from baseline to month 24/30.
10. Brain atrophy as defined by the percentage of change from baseline to month 12, from month 12 to month 24/30 and from baseline to month 24/30 in brain volume.
11. Serum samples are collected from all subjects in order to investigate the potential mechanism of action of laquinimod and additional biomarkers of inflammation and potential biomarkers of MS disease. These samples are collected at months: 0 (baseline), 1, 12 and 24 (even if the study is extended to month 30).
12. Population PK - fitness of a population model to different covariates is evaluated. (covariates such as: gender, age, concomitant medications, weight, AE profile, habits).
13. The change from baseline to month 24/30 (termination/early discontinuation) in binocular visual acuity, as assessed by the number of letters read correctly from 2 meters distance on 100%, 2.5% and 1.25% contrast level Sloan letter/Tumbling-E charts.

The analysis of the total number of confirmed relapses during the study period is based on baseline adjusted Quasi-Likelihood (over-dispersed) Poisson Regression.

The analysis of the accumulation of physical disability is based on Cox's Proportional Hazard model.

The analysis of MSFC is based on baseline-adjusted Analysis of Covariance.

The analysis of the secondary MRI endpoints is based on baseline-adjusted Negative Binomial Regression.

### Results

This study assesses the efficacy, tolerability and safety of daily dose of 0.6 mg laquinimod as compared to placebo in RRMS subjects.

Daily oral administration of 0.6 mg laquinimod reduces the number of confirmed relapses and therefore the relapse rate, in relapse-remitting multiple sclerosis patients during the double blind study period.

Daily oral administration of 0.6 mg laquinimod also reduces the accumulation of physical disability in relapse-remitting multiple sclerosis patients, as measured by the time to confirmed progression of EDSS.

Daily oral administration of 0.6 mg laquinimod also reduces MRI-monitored disease activity in relapse-remitting multiple sclerosis patients, as measured by the cumulative number of enhancing lesions on T₁-weighted images, the cumulative number of new hypointense lesions on T₁-scans, and the cumulative number of new T₂ lesions.

### EXAMPLE 2: Clinical Trial (Phase III) - Benefit-Risk Assessment of Avonex® and Laquinimod

A multinational, multicenter, randomized, parallel-group, clinical trial is performed in subjects with RRMS ("BRAVO"). BRAVO is conducted to assess the efficacy, safety and tolerability of laquinimod over placebo in a double-blinded design and of a reference arm of Interferon β-1a (Avonex®) in a rater-blinded design.

The 2006 EMEA Guidelines for MS clinical trials states that active control parallel group trials comparing the new treatment to an already approved treatment are needed in order to give the comparative benefit/risk ratio of the new treatment, at least in those treatment intended to prevent relapses. Three-arm studies with placebo, test product and active control are a preferred design.

Avonex® (Interferon beta-1a) is a 166-amino acid glycoprotein produced by recombinant DNA technology using genetically engineered Chinese Hamster ovary cells into which the human interferon beta gene has been introduced. The amino acid sequence of Avonex® is identical to that of natural human interferon beta.

Avonex® is a marketed drug indicated for the treatment of patients with relapsing forms of MS to slow the accumulation of physical disability and decrease the frequency of clinical exacerbations. Patients with multiple sclerosis in whom efficacy has been demonstrated include patients who have experienced a first clinical episode and have MRI features consistent with MS.

The recommended dosage of Avonex® is 30mcg injected intramuscularly once a week.

### Study Title

A multinational, Multicenter, Randomized, Parallel-Group study performed in subjects with Relapsing-Remitting Multiple Sclerosis (RRMS) to assess the efficacy, safety and tolerability of laquinimod over placebo in a double-blind design and of a reference arm of Interferon β-1a (Avonex®) in a rater-blinded design.

### Study Duration

Screening phase: 1 month.

Treatment phase: 24 months of once-daily oral administration of laquinimod 0.6 mg, matching oral placebo or once-weekly intramuscular administration of Interferon β-1a (Avonex®) 30 mcg.

### Study Population

Subjects with RRMS.

### Study Design

Eligible subjects are randomized in a 1:1:1 ratio (oral laquinimod: oral placebo: Avonex®) and assigned to one of the following three treatment arms:
1. Laquinimod 0.6 mg per os once daily (400 subjects). The 0.6 mg laquinimod is administered as laquinimod capsules which contains 0.6 mg of Laquinimod Acid per capsule with meglumine.
2. Matching placebo (for laquinimod) per os once daily (400 subjects).
3. Interferon β-1a (Avonex®) 30 mcg intramuscular injection once weekly (400 subjects).

Subjects on oral treatment are managed in a double-blind manner. Subjects assigned to injectable treatment with Avonex® and their Treating Neurologist/Physician are unblinded to the treatment assignment, but assessed neurologically by an Examining Neurologist/Physician in a blinded manner (potential IM injection sites are covered).

During the treatment phase, subjects are evaluated at study sites for a total of 12 scheduled visits at months: -1 (screening), 0 (baseline), 1, 2, 3, 6, 9, 12, 15, 18, 21 and 24 (termination/early discontinuation).

Subjects successfully completing the study are offered the opportunity to enter into a 1-year open-label extension in which laquinimod 0.6 mg/d are administered.

During the study, the following assessments are performed (regardless of the treatment assignment) at the specified time points:
1. Vital signs are measured at each study visit.
2. A physical examination is performed at months -1 (screening), 0 (baseline) 1, 3, 6, 12, 18 and 24 (termination/early discontinuation).
3. The following safety clinical laboratory tests are performed:
   a. Complete blood count (CBC) with differential - at all scheduled visits. A reticulocyte count is added to the CBC at months 0 (baseline) and 24 (termination/early discontinuation) as well as in occasions of significant decrease in hemoglobin.
   b. Serum chemistry (including electrolytes, liver enzymes, direct and total bilirubin, CPK and pancreatic amylase), and urinalysis - at all scheduled visits.
   c. Serum TSH, T3 and Free T4 are measured at months 0 (baseline), 6, 12, 18 and 24 (termination/early discontinuation).
   d. A rapid urine β-hCG test is performed in women of child-bearing potential at baseline (month 0; all subjects) and at each scheduled study visit thereafter (at site; only subjects assigned to oral treatment).
   e. β-hCG in women of child-bearing potential are performed at each study visit.
   f. Starting after visit Month 3 a rapid urine β-hCG test is performed in women of child-bearing potential (only those assigned to oral treatment) every 28 (±2) days. The subject is contacted by telephone with 72 hours after the test is scheduled to be performed and asked specific questions regarding the test. In case of suspected pregnancy (positive urine β-hCG test result), the caller makes sure that the study drug has been discontinued and the subject is instructed to arrive to the site as soon as possible with all study drugs.
4. Markers of inflammation (serum conventional C-reactive protein and fibrinogen) are measured at all scheduled visits.
5. Serum samples are collected for evaluation of immunological parameters and response to treatment with either laquinimod or Avonex®, as well as further investigation of the potential mechanisms of action of laquinimod or for the detection of infectious agents. These samples are collected at months 0, 12 and 24.
6. During the first 3 months of the study, periodical phone calls are placed by the site personnel every two weeks. A list of predefined questions relating to signs/symptoms suggestive of vascular thrombosis is presented to the subject. In case of suspected thrombotic event, the subject is requested to arrive at the site immediately for further evaluation.
7. ECG is performed at months -1 (screening; additional recording, up to 30 minutes apart are performed if QTc is > 450 msec), 0 (baseline; three recordings, 15 minute apart), 1, 2, 3, 6, 12, 18 and 24 (termination/early discontinuation).
8. Chest X-ray is performed at month -1 (screening) (if not performed within 6 months prior to screening visit).
9. Adverse Events (AEs) are monitored throughout the study.
10. Concomitant medications are monitored throughout the study.
11. Neurological evaluations, including Neurostatus [Functional Systems (FS), Expanded Disability Status Scale (EDSS; Converted scale), Ambulation Index (AI)] and Timed-25 foot walk test are performed at months -1 (screening), 0 (baseline) and every 3 months thereafter, until termination/early discontinuation. (At screening visit, the Timed-25 foot walk test is performed 3 times, for practicing purposes, as a part of the MSFC).
12. MS Functional Composite (MSFC) is assessed at months -1 (screening) (three practices for training purposes only), 0 (baseline), 6, 12, 18 and 24 (termination/early discontinuation).
13. The general health status is assessed by the EuroQoL (EQ5D) questionnaire at months 0 (baseline) and 24 (termination/early discontinuation).
14. The general health status and quality of life parameters are assessed by the Short-Form general health survey (SF-36) subject-reported questionnaire at month 0 (baseline) and every 6 months thereafter until termination/early discontinuation, inclusive.
15. Subject-reported fatigue is assessed by the Modified Fatigue Impact Scale (MFIS) at months 0 (baseline), 2, 6, 12, 18 and 24 (termination/early discontinuation).
16. All subjects undergo 3 MRI scans at months 0 (13-7 days prior to baseline visit), 12 and 24 (termination/early discontinuation).
17. All subjects undergo 5 assessment of binocular low-contrast visual acuity using the 1.25%, 2.5% and 100% contrast level charts [Sloan letter or Tumbling-E] in each assessment, at months 0 (baseline), 6, 12, 18 and 24 (termination/early discontinuation).
18. Blood test for Factor V Leiden mutation is performed at screening visit.
19. Serologies for Hepatitis B and C viruses are performed at screening visit.
21. Relapses are confirmed/monitored throughout the study. Since the "in study" relapse definition must be supported by an objective neurological evaluation, a neurological deficit must sustain long enough to eliminate pseudo-relapses. Therefore, in Experiment 2, a confirmed relapse is the appearance of one or more new neurological abnormalities or the reappearance of one or more previously observed neurological abnormalities wherein the change in clinical state lasts at least 48 hours and is immediately preceded by an improving neurological state of at least thirty (30) days from onset of previous relapse.
20. The allowed treatment for a relapse is intravenous methylprednisolone 1 gr/day for up to 5 consecutive days.

### Re-consent criteria

In case of a confirmed diagnosis of MS relapse (as defined in the protocol), or in case of an increase in EDSS of ≥2.0 points, sustained for ≥3 months, the following actions are taken:
1. The subject is reminded of the current available MS medications/treatments and the opportunity to terminate the study.
2. The subject is requested to re-sign an informed consent form if he/she chooses to continue to participate in the study, in the same treatment assignment.

### Monitoring

Safety monitoring plan and stopping rules are set in place for the management of: 1) elevated liver enzymes, 2) inflammatory events, 3) thrombotic events and 4) pancreatitis.

### Ancillary studies

Pharmacogenetic (PGt) assessment: Upon the approval of this ancillary study by EC/IRB, blood samples for PGt parameters are collected from all subjects who signed the informed consent form at month 0 (baseline).

Relationship between PGt and response to laquinimod or to Avonex® in terms of clinical, MRI and safety parameters is assessed in all sites.

The effect of general health and symptom severity on work is assessed by the work productivity and activities impairment - General Health (WPAI-GH) questionnaire at month 0 (baseline) and every 3 months thereafter, until month 24 (termination/early discontinuation) visit (this assessment is performed in all subjects from the U.S. sites only).

### Number of Subjects

Approximately 1200 subjects.

Prior to the end of the recruitment period, a blinded relapse rate and sample size reassessment is performed. Based on the newly estimated relapse rate of the population, the sample size may be increased.

### Inclusion/Exclusion Criteria

### Inclusion Criteria

1. Subjects must have a confirmed and documented MS diagnosis as defined by the Revised McDonald Criteria [Ann Neurol 2005:58:840-846], with a relapsing-remitting disease course.
2. Subjects must be ambulatory with Converted EDSS score of 0-5.5 in both screening and baseline visits.
3. Subjects must be in a stable neurological condition and free of corticosteroid treatment [intravenous (IV), intramuscular (IM) and or per os (PO)] 30 days prior to screening (month -1) and between screening (month -1) and baseline (month 0) visits.
4. Subjects must have had experienced one of the following:
   a. At least one documented relapse in the 12 months prior to screening, or
   b. At least two documented relapses in the 24 months prior to screening, or
   c. One documented relapse between 12 and 24 months prior to screening with at least one documented T1-Gd enhancing lesion in an MRI performed within 12 months prior to screening.
5. Subjects must be between 18 and 55 years of age, inclusive.
6. Women of child-bearing potential must practice an acceptable method of birth control. Acceptable methods of birth control in this study include: surgical sterilization, intrauterine devices, oral contraceptive, contraceptive patch, long-acting injectable contraceptive, partner's vasectomy or a double-barrier method (condom or diaphragm with spermicide).
7. Subjects must be able to sign and date a written informed consent prior to entering the study.
8. Subjects must be willing and able to comply with the protocol requirements for the duration of the study.

### Exclusion Criteria

1. An onset of relapse or any treatment with corticosteroid (intravenous [IV], intramuscular [IM] and/or per os [PO]) or ACTH between month -1 (screening) and 0 (baseline).
2. Subjects with progressive forms of MS.
3. Use of experimental or investigational drugs, and/or participation in drug clinical studies within the 6 months prior to screening.
4. Use of immunosuppressive (including Mitoxantrone (Novantrone®)) or cytotoxic agents within 6 months prior to the screening visit.
5. Previous use of either of the following: natalizumab (Tysabri®), cladribine, laquinimod, Interferon beta-1a (Avonex® or Rebif®), Interferon beta beta-1b (Betaseron®/Betaferon®) or any other experimental Interferon-beta for MS.
6. Previous treatment with glatiramer acetate (Copaxone®) or IVIG within 2 months prior to screening visit.
7. Chronic (more than 30 consecutive days) systemic (IV, PO or IM) corticosteroid treatment within 2 months prior to screening visit.
8. Previous total body irradiation or total lymphoid irradiation.
9. Previous stem-cell treatment, autologous bone marrow transplantation or allogenic bone marrow transplantation.
10. A known history of tuberculosis.
11. Acute infection within 2 weeks prior to baseline visit.
12. Major trauma or surgery within 2 weeks prior to baseline visit.
13. Known human immunodeficiency virus (HIV) positive status.
14. Use of inhibitors of CYP3A4 within 2 weeks prior to baseline visit.
15. Use of amiodarone within 2 years prior to screening visit.
16. Pregnancy or breastfeeding.
17. A ≥3xULN serum elevation of either ALT or AST at screening.
18. Serum direct bilirubin which is ≥2xULN at screening.
19. A QTc interval which is > 450 msec (according machine output), obtained from:
   a. Two ECG recordings at screening visit, or
   b. The mean value calculated from 3 baseline ECG recordings.
20. Subjects with a clinically significant or unstable medical or surgical condition that, in the Investigator's opinion, would preclude safe and complete study participation, as determined by medical history, physical examination, ECG, laboratory tests or chest or chest X-ray. Such conditions may include:
   a. A cardiovascular or pulmonary disorder that cannot be well-controlled by standard treatment permitted by the study protocol.
   b. A gastrointestinal disorder that may affect the absorption of study medication.
   c. Renal, metabolic or hematological diseases.
   d. Thyroid disease: a subject with hyperthyroidism is not permitted to participate in the study. A subject with hypothyroidism may be permitted to participate in the study provided that he/she is clinically euthyroid and considered stable.
   e. Liver disease, such as cirrhosis.
   f. A family history of Long-QT syndrome.
   g. A history of drug and/or alcohol abuse.
   h. A current major psychiatric disorder, including schizophrenia or severe depression, with or without suicidal ideation.
   i. A history of seizure disorder, with the last convulsion occurring within 12 months prior to screening visit.
21. A known history of sensitivity to Gadolinium.
22. Inability to successfully undergo MRI scanning.
23. A known drug hypersensitivity that would preclude administration of laquinimod, such as hypersensitivity to: mannitol, meglumine or sodium stearyl fumarate.
24. A known history of hypersensitivity to natural or recombinant interferon beta, human albumin, or any other component of the formulation of Avonex®.

### Route and Dosage Form

Laquinimod arm: one capsule containing laquinimod 0.6 mg is administered orally once daily. The 0.6 mg laquinimod capsule contains 0.6 mg of Laquinimod Acid per capsule with meglumine.

The 0.6 mg laquinimod capsule is manufactured according to the method disclosed in PCT International Application Publication No. WO/2007/146248, published December 21, 2007 (see, page 10, line 5 to page 11, line 3).

Matching placebo for laquinimod arm: one capsule is administered orally once daily.

Avonex® arm: one injection of Interferon β-1a (Avonex®) 30 mcg is administered intramuscularly once weekly.

### Outcome Measures

### Primary Outcome Measure

The number of confirmed relapses during the treatment period.

### Secondary Outcome Measures

Type-I error is controlled by employing the Hierarchical Approach, (i.e. each endpoint is analyzed only in case the preceding endpoint has a p-value less or equal to 0.05 for laquinimod 0.6 mg over placebo comparison) according to the following order:
1. Disability, as assessed by the MSFC score at the end of the treatment period.
2. Brain atrophy as defined by the percent brain volume change from baseline at the end of the treatment period.
3. Accumulation of physical disability measured by the time to confirmed progression of EDSS (A confirmed progression of EDSS is defined as a 1 point increase from baseline on EDSS score if baseline was between 0 and 5.0, or a 0.5 point increase if baseline EDSS was 5.5, confirmed 3 months later. Progression cannot be confirmed during a relapse).

### Safety and Tolerability Outcome Measures

1. Adverse events.
2. Vital signs.
3. ECG findings.
4. Clinical laboratory parameters.
5. Proportion of subjects (%) who prematurely discontinued from the study, reason of discontinuation and the time to withdrawal.
6. Proportion of subjects (%) who prematurely discontinued from the study due to AEs and the time to withdrawal.

### Benefit/Risk Assessment

The Avonex (RP reference arm is compared to the placebo treatment group with respect to the same endpoints as for the comparison between the laquinimod group and the placebo group.

These endpoints include:
1. The number of confirmed relapses during the treatment period.
2. Disability measures based on EDSS and MSFC neurological scales.
3. MRI parameters.
4. Safety as assessed by adverse events, vital signs, ECG and clinical laboratory parameters.
5. Tolerability
6. Quality of life scales such as: Modified Fatigue Impact Scale (MFIS), General health status, as assessed by the EuroQoL (EQ5D) questionnaire and the Short-Form general Health survey (SF-36) subject-reported questionnaire.

The comparative assessment of the benefit/risk ratio between the two active arms (laquinimod and Avonex®) is based on the following aspects:
1. Efficacy parameters (Disability, MRI parameters, other relapse-related endpoints).
2. Safety and tolerability.
3. Quality of life.

### Additional Exploratory Endpoints

The following assessments are presented in an exploratory manner:
1. The total number of enhancing lesions on T1-weighted images taken at months 12 and 24 (termination/early discontinuation).
2. The number of enhancing lesions on a T1-weighted image taken at month 12.
3. The number of enhancing lesions on a T1-weighted image taken at month 24 (termination/early discontinuation).
4. The total number of new hypointense lesions ("black holes") on enhanced T1 scans taken at months 12 and 24 (termination/early discontinuation).
5. The total number of new hypointense lesions ("black holes") on an enhanced T1 scan taken at month 12.
6. The total number of new hypointense lesions ("black holes") on an enhanced T1 scan taken at month 24 (termination/early discontinuation).
7. The total number of new/newly enlarging T2 lesions on scans taken at months 12 and 24 (termination/early discontinuation).
8. The number of new/newly enlarging T2 lesions on a scan taken at month 12.
9. The total number of new/newly enlarging T2 lesions on scans taken at month 24 (termination/early discontinuation).
10. The change in T2-lesion volume between months 0 (baseline) and 24 (termination/early discontinuation).
11. The volume of T-2 lesions at termination/early discontinuation of treatment period.
12. The change from baseline to month 24 (termination/early discontinuation) in the volume of hypointense lesions on enhanced T1 scans.
13. Brain atrophy as defined by the percent brain volume change from: 1) baseline to month 12 and b) month 12 to month 24 (termination/ early discontinuation).
14. The change from baseline to month 24 (termination/early discontinuation) in binocular visual acuity, as assessed by the number of letters read correctly from 2 meters distance on 1.25%, 2.5% and 100% contrast level Sloan letter/Tumbling-E charts.
15. Subject-reported fatigue, as assessed by the Modified Fatigue Impact Scale (MFIS).
16. The time to the first confirmed relapse during the treatment period.
17. The proportion of relapse-free subjects.
18. The rate of confirmed relapses during the treatment period requiring hospitalization and/or IV steroids.
19. The general health status, as assessed by the EuroQoL (EQ5D) questionnaire.
20. The general health status and health-related quality of life, as assessed by the Short-Form general health survey (SF-36) subject-reported questionnaire.

### Results

This study assesses the efficacy, safety and tolerability of laquinimod over placebo in a double-blind design and of a reference arm of Interferon β-1a (Avonex®), in a rater-blinded design and to perform a comparative benefit/risk assessment between oral laquinimod and injectable Interferon β-1a (Avonex®).

Daily oral administration of 0.6 mg laquinimod reduces the number of confirmed relapses, which is directly related to the relapse rate, in relapse-remitting multiple sclerosis patients.

Daily oral administration of 0.6 mg laquinimod also reduces the accumulation of physical disability in relapse-remitting multiple sclerosis patients, as measured by the MSFC score at the end of the treatment period, and time to confirmed progression of EDSS.

Daily oral administration of 0.6 mg laquinimod also reduces brain atrophy in relapse-remitting multiple sclerosis patients, as measured by percent brain volume change from baseline to at the end of the treatment period.

### References

1. PCT International Application Publication No. WO 2007/047863, published April 26, 2007, international filing date October 18, 2006.
2. PCT International Application Publication No. WO 2007/146248, published December 21, 2007, international filing date June 12, 2007.
3. Barkhof, F. (1999) "MRI in Multiple Sclerosis: Correlation with Expanded Disability Status Scale (EDSS)", Multiple Sclerosis. 5(4):283-286 (Abstract).
4. Bjartmar C, Fox RI. (2002) "Pathological mechanisms and disease progression of multiple sclerosis: therapeutic implication", Drugs of Today. 38:7-29.
5. Brex PA, et al., (2002) "A longitudinal study of abnormalities on MRI and disability from multiple sclerosis", N Engl J Med. Jan 17, 2002 346(3):158-64.
6. Brunmark C, et al., (2002) "The new orally active immunoregulator laquinimod (ABR-215062) effectively inhibits development and relapses of experimental autoimmune encephalomyelitis", J Neuroimmunology. 130:163-172.
7. Comi G, et al. (2007) LAQ/5062 Study Group. "The Effect of Two Doses of Laquinimod on MRI-Monitored Disease Activity in Patients with Relapsing-Remitting Multiple Sclerosis: A Multi-Center, Randomized, Double-Blind, Placebo-Controlled Study", Presented at: 59th Annual Meeting of the American Academy of Neurology; April 28-May 5, 2007; Boston, MA.
8. De Stefano N, Narayanan S, Pelleties D, Grancis GS, Antel J. (1999) "Evidence of early axonal damage in patients with multiple sclerosis", Neurology. 52(Suppl 2):A378.
9. Martin Dunitz. Multiple sclerosis therapeutics, Ed. Rudick and Goodkin. London: Taylor & Francis, 1999.
10. Durelli L, Verdun E, Barbero P, Bergui M, Versino E, Ghezzi A, Montanari E, Zaffaroni M, and the Independent Comparison of Interferon (INCOMIN) Trial Study Group. (2002) "Every-other-day interferon beta-1b versus once-weekly interferon beta-1a for multiple sclerosis: results of a 2-year prospective randomised multicentre study (INCOMIN)", Lancet. 359:1453-60.
11. EMEA Guideline on Clinical Investigation of Medicinal Products for the Treatment of Multiple Sclerosis (CPMP/EWP/561/98 Rev. 1, Nove.2006).
12. EPAR, Rebif®, Scientific Discussion.
13. Fischer JS et al., (1999) "The Multiple Sclerosis Functional Composite measure (MSFC): an integrated approach to MS clinical outcome assessment" Multiple Sclerosis, 5(4):244-250.
14. Fisk JD et al., (1994) "The Impact of Fatigue on Patients with Multiple Sclerosis", Can J Neurol Sci. 21:9-14.
15. Fisk JD et al., (1994) "Measuring the Functional Impact of Fatigue: Initial Validation of Fatigue Impact Scale", Clin Inf Dis. 18 Suppl 1:S79-83.
16. Frohman EM et al., (2003) "The utility of MRI in suspected MS: report of the Therapeutics and Technology Assessment Subcommittee of the American Academy of Neurology", Neurology. Sep 9, 2003, 61 (5) : 602-11.
17. Golder W, (2007) "Magnetic resonance spectroscopy in clinical oncology", Onkologie. 27(3): 304-9.
18. Grossman RI et al., (1994) Magnetization transfer: theory and clinical applications in neuroradiology", RadioGraphics. 14:279-290.
19. Hartung HP, Munschauer F, Schellekens H. (2005) "Significance of neutralizing antibodies to interferon beta during treatment of multiple sclerosis: expert opinions based on the Proceedings of an International Consensus Conference", Eur J Neurol. 12:588-601.
20. Hauser SL, et al. (1983) "Intensive immunosuppression in progressive multiple sclerosis", New Engl J Med. 308:173-180.
21. Hohlfeld R, Kerschensteiner M, Stadelmann C, Lassmann H, Wekerle H. (2000) "The neuroprotective effect of inflammation: implications for the therapy of multiple sclerosis", J Neuroimmunol. 107:161-166.
22. Kurtzke JF, (1983) "Rating neurologic impairment in multiple sclerosis: an expanded disability status scale (EDSS)", Neurology 33(11):1444-1452.
23. Lublin FD, Reingold SC (1996) "Defining the clinical course of multiple sclerosis", Neurol. 46:907-911.
24. Mehta RC, et al. (1996) "Magnetization transfer magnetic resonance imaging: a clinical review", Topics in Magnetic Resonance Imaging 8(4):214-30.
25. McDonald, (2001) "Guidelines from the International Panel on the Diagnosis of Multiple Sclerosis" Ann. Neurol. 50:121-127.
26. Miki, Y, et al. (1999) "Relapsing-Remitting Multiple Sclerosis: Longitudinal Analysis of MR Images - Lack of Correlation between Changes in T2 Lesion Volume and Clinical Findings", Radiology. 213:395-399.
27. Neuhaus O, Archelos JJ, Hartung HP. (2003) "Immunomodulation in multiple sclerosis: from immunosuppression to neuroprotection", Trends Pharmacol Sci. 24:131-138.
28. Noseworthy JH, Lucchinetti C, Rodriguez M, Weinshenker BO. (2000) "Multiple sclerosis", N Engl J Med. 343:938-952.
29. Panitch H, Goodin DS, Francis G, Chang P, Coyle PK, O'Connor P, Monaghan E, Li D, Weinsjenker B, for the EVIDENCE (Evidence of Interferon Dose-response: European North American Comparative Efficacy) Study Group and the University of British Columbia MS/MRI Research Group. (2002) "Randomized comparative study of interferon β-1a treatment regiments in MS", The EVIDENCE Trial. Neurology. 59:1496-1506.
30. Polman, C. et al., (2005) "Diagnostic criteria for multiple sclerosis: 2005 revisions to the McDonald Criteria", Annals of Neurology, Volume 58 Issue 6, Pages 840 - 846.
31. Polman, C. et al., (2005) "Treatment with laquinimod reduces development of active MRI lesions in relapsing MS", Neurology. 64:987-991.
32. Poser CM. Paty DW, Scheinberg L, et al. (1983) "New Diagnostic Criteria for Multiple Sclerosis: Guidelines for Research Protocols", Annals of Neurology, March 1983, 13(3):227-230.
33. Rosen Y, (2007) "The Recent advances in magnetic resonance neurospectroscopy", Neurotherapeutics. 27 (3) : 330-45.
34. Rudick, R. (1999) "Disease-Modifying Drugs for Relapsing-Remitting Multiple Sclerosis and Future Directions for Multiple Sclerosis Therapeutics", Neurotherpatueics. 56:1079-1084.
35. Runström A, et al. (2002) "Laquinimod (ABR-215062) a candidate drug for treatment of Multiple Sclerosis inhibits the development of experimental autoimmune encephalomyelitis in IFN-β knock-out mice", (Abstract), Medicon Valley Academy, Malmoe, Sweden.
36. Sandberg-Wollheim M, et al. (2005) "48-week open safety study with high-dose oral laquinimod in patients", Mult Scler. 11:S154 (Abstract).
37. Sorenson PS. (2006) "Neutralising antibodies to interferon-β - measurement, clinical relevance, and management", J Neurol. 253[Suppl 6]:VI/16-VI/22.
38. The National MS Society (USA), The Disease Modifying Drug Brochure, October 19, 2006.
39. Yang J-S, et al., (2004) "Laquinimod (ABR-215062) suppresses the development of experimental autoimmune encephalomyelitis, modulates the Th1/Th2 balance and induces the Th3 cytokine TGF-β in Lewis rats", J. Neuroimmunol. 156:3-9.
40. Zou LP, et al. (2002) "Suppression of experimental autoimmune neuritis by ABR-215062 is associated with altered Th1/Th2 balance and inhibited migration of inflammatory cells into the peripheral nerve tissue", Neuropharmacology. 42:731.

The invention may be characterised by the following clauses;
1. A method of reducing the relapse rate in a relapsing-remitting multiple sclerosis human patient, the method comprising orally administering to the patient laquinimod or a pharmaceutically acceptable salt thereof at a daily dose of 0.6 mg laquinimod so as to thereby reduce the relapse rate.
2. The method clause 1, wherein the relapse rate is reduced by at least 30%.
3. The method clause 2, wherein the relapse rate is reduced by at least 70%.
4. The method of any one of clauses 1-3, wherein the laquinimod is administered in the form of laquinimod sodium.
5. The method of any one of clauses 1-4, wherein the laquinimod is administered as monotherapy for relapsing-remitting multiple sclerosis.
6. The method of any one of clauses 1-4, wherein the laquinimod is administered as adjunct therapy with an other relapsing-remitting multiple sclerosis treatment.
7. The method of clause 6, wherein the other relapsing-remitting multiple sclerosis treatment is administration of interferon beta 1-a, interferon beta 1-b, glatiramer acetate, mitoxantrone or natalizumab.
8. The method of any one of clauses 1-7, wherein the administration is for a period of greater than 24 weeks.
9. A method of reducing the accumulation of physical disability in a relapsing-remitting multiple sclerosis human patient, the method comprising orally administering to the patient laquinimod or a pharmaceutically acceptable salt thereof at a daily dose of 0.6 mg laquinimod so as to thereby reduce the accumulation of physical disability.
10. The method of clause 9, wherein the accumulation of physical disability is assessed by the time to confirmed disease progression as measured by Kurtzke Expanded Disability Status Scale (EDSS) score.
11. The method of clause 10, wherein the patient had an EDSS score of 0-5.5 prior to administration of laquinimod.
12. The method of clause 10, wherein the patient had an EDSS score of 5.5 or greater prior to administration of laquinimod.
13. The method of clause 11, wherein confirmed disease progression is a 1 point increase of the EDSS score.
14. The method of clause 12, wherein confirmed disease progression is a 0.5 point increase of the EDSS score.
15. The method of any one of clauses 9-14, wherein time to confirmed disease progression is increased by 20-60%.
16. The method of clause 15, wherein time to confirmed disease progression is increased by 50%.
17. The method of any one of clauses 9-16, wherein the laquinimod is administered in the form of laquinimod sodium.
18. The method of any one of clauses 9-17, wherein the laquinimod is administered as monotherapy for relapsing-remitting multiple sclerosis.
19. The method of any one of clauses 9-17, wherein the laquinimod is administered as adjunct therapy with an other relapsing-remitting multiple sclerosis treatment.
20. The method of clause 19, wherein the other relapsing-remitting multiple sclerosis treatment is administration of interferon beta 1-a, interferon beta 1-b, glatiramer acetate, mitoxantrone or natalizumab.
21. The method of any one of clauses 9-20, wherein the administration is for a period of greater than 24 weeks.
22. A pharmaceutical oral unit dosage form of 0.6 mg laquinimod for use in reducing the relapse rate in a relapsing-remitting multiple sclerosis human patient.
23. A pharmaceutical oral unit dosage form of 0.6 mg laquinimod for use in reducing the accumulation of physical disability in a relapsing-remitting multiple sclerosis human patient.

## Claims

1. A pharmaceutical oral unit dosage form of 0.6 mg laquinimod for use in reducing the relapse rate and/or reducing the accumulation of physical disability in a relapsing multiple sclerosis human patient.

2. The pharmaceutical oral unit dosage form of claim 1, wherein the relapsing multiple sclerosis is relapsing-remitting multiple sclerosis.

3. The pharmaceutical oral unit dosage form of claims 1 or 2, wherein the accumulation of physical disability is assessed by the time to confirmed disease progression.

4. The pharmaceutical oral unit dosage form of claims 1 or 2, wherein the time to confirmed disease progression is measured by Kurtzke Expanded Disability Status Scale (EDSS) score.

5. The pharmaceutical oral unit dosage form of any one of claims 1-4 for use in reducing the accumulation of physical disability in a patient having an EDSS score of 0-5.5.

6. The pharmaceutical oral unit dosage form of any one of claims 1-4 for use in reducing the accumulation of physical disability in a patient having an EDSS score of 5.5 or greater.

7. The pharmaceutical oral unit dosage form of claim 5, wherein confirmed disease progression is a 1 point increase of the EDSS score.

8. The pharmaceutical oral unit dosage form of claim 6, wherein confirmed disease progression is a 0.5 point increase of the EDSS score.

9. The pharmaceutical oral unit dosage form of any one of claims 4-8, wherein time to confirmed disease progression is increased by 20-60%.

10. The pharmaceutical oral unit dosage form of claim 9, wherein time to confirmed disease progression is increased by 50%.

11. The pharmaceutical oral unit dosage form of any one of claims 1-10, wherein the laquinimod is in the form of laquinimod sodium.

12. The pharmaceutical oral unit dosage form of any one of claims 1-11, for use as a monotherapy for relapsing-remitting multiple sclerosis.

13. The pharmaceutical oral unit dosage form of any one of claims 1-11, for use as an adjunct therapy with an other relapsing-remitting multiple sclerosis treatment.

14. The pharmaceutical oral unit dosage form of claim 13, wherein the other relapsing-remitting multiple sclerosis treatment is administration of interferon beta 1-a, interferon beta 1-b, glatiramer acetate, mitoxantrone or natalizumab.

15. The pharmaceutical oral unit dosage form of any one of claims 1-14 prepared for daily use for a period of greater than 24 weeks or for a period of 24 months or more.
